# EUROPEAN PATENT SPECIFICATION

(11) **EP 1 857 494 B1**
(45) Date of publication and mention of the grant of the patent: **26.06.2013**
(21) Application number: 06713668.9
(22) Date of filing: 14.02.2006
(51) Int. Cl.: C08J 3/24, A61K 47/42, C08J 3/28, A61L 27/00, A61L 27/22, A61L 27/52, C08L 101/14, A61K 9/00, C08J 3/075

(54) **HYDROGEL FOR MEDICAL USE**
HYDROGEL ZUR MEDIZINISCHEN VERWENDUNG
HYDROGEL DESTINE A UN USAGE MEDICAL

(30) Priority: 14.02.2005 JP 2005036309
(43) Date of publication of application: 21.11.2007
(73) Proprietor: Medgel Corporation, Kyoto-shi, Kyoto 612-8043 (JP)
(72) Inventor: TABATA, Yasuhiko, Kyoto, 6110024 (JP); ASAHARA, Tomohiko, Shiga, 5250023 (JP)
(74) Representative: Vossius & Partner
(86) International application number: PCT/JP2006/302527
(87) International publication number: WO 2006/085653

(56) References cited:
- EP-A1- 0 702 959
- EP-A1- 1 415 663
- WO-A2-02/085419
- JP-A- 03 259 927
- JP-A- 60 008 326
- JP-A- 2001 008 634
- EINERSON N J ET AL: "Synthesis and physicochemical analysis of gelatin-based hydrogels for drug carrier matrices", BIOMATERIALS, ELSEVIER SCIENCE PUBLISHERS BV., BARKING, GB, vol. 24, no. 3, 1 February 2003 (2003-02-01), pages 509-523, XP004390671, ISSN: 0142-9612, DOI: DOI:10.1016/S0142-9612(02)00369-1

## Description

The present invention relates to a medical hydrogel that, through utilization of its degradation characteristics in vivo, is well-adapted for use for drug delivery, for adhesion prevention, for wound healing, and so forth.

### Background Of The Invention

A number of medical hydrogels are currently known, for example, carboxymethyl cellulose, alginic acid, pectin, hyaluronic acid, dextran sulfate, collagen, chitosan, polylysine, polyallylamine, and so forth, and many of them can also be used implanted in a body. While some of them have been proposed and are employed as wound dressing materials, lubricating materials, and adhesion preventing materials, their function and properties are not necessarily easy to control, and it is known to be quite difficult to obtain a medical hydrogel that is completely adapted to the intended use. For example, even when the attempt is made to control the physical properties of hyaluronic acid by setting its molecular weight and/or degree of crosslinking, this is a very limited avenue. In addition it is almost impossible to control the in vivo kinetics of a hyaluronic acid hydrogel. Moreover, hydrogels whose physical properties can be easily controlled lack excretability and suffer from a poor or unsatisfactory degradability, and it is thus not easy from a technical standpoint to obtain a hydrogel adapted to a particular objective. Furthermore, when such hydrogels are impregnated with a drug with the goal of using the hydrogel as a vehicle for drug delivery, there is the fundamental problem that the drug generally elutes easily from the gel, which makes it impossible to carry out delivery at a desired rate, and as a consequence hydrogels are not being used for this application.

The conventional technology related to drug delivery includes the introduction of the drug to be delivered into a solid or capsule-type carrier comprising a bioabsorbable synthetic polymer compound and release of the drug along with the degradation and/or absorption of the polymer. This technology depends on the in vivo hydrolysis of the polymer backbone, and the release rate is thus governed by the type and structure of the polymer compound. As a consequence, a controlled release and kinetics appropriate to a desired release interval cannot be expected. In addition, the carrier is limited to a solid form, which impairs unrestricted selection of the location and method of delivery. There have also been limitations on the type of drug that can be delivered by this method. With regard to other applications of hydrogels, they are in use for wound dressings; however, no technology exists that can produce a hydrogel that is simultaneously satisfactory with regard to a desired water content and physical properties, and thus it has not been possible to devise a shape stabilization accommodative of the delivery interval. A number of problems also occur with the sterilization procedure and method of production in the case of high water content hydrogels.

The following is a document related to the present invention: WO94/27630.

### SUMMARY OF THE INVENTION

The present invention was achieved based on the circumstances described above. An object of the present invention is to provide a medical hydrogel that has properties appropriate to the intended use. With regard to the application of a hydrogel as a vehicle for drug delivery, a particular object of the present invention is to provide a hydrogel that has the ability to reliably provide a continuous delivery interval.

The present invention provides a hydrogel in which the medium is substantially water, or a dried material thereof, that is obtained by crosslinking a gelatin having 20 to 30 amino groups per molecule and the same or greater number of carboxyl groups per molecule or a gelatin derivative having 2 to 100 amino groups per molecule and 50 or more carboxyl groups per molecule, and that has at least 0.010 but no more than 50 crosslink points per molecule.

The medical hydrogel of the present invention is preferably obtained by crosslinking the gelatin or a derivative thereof by one or more methods selected from chemical methods and physical methods. Preferably, the medical hydrogel of the present invention or dry material thereof is crosslinked by a physical method and has a degree of crosslinking that varies in accordance with a distance from the surface, for example, in the direction of the thickness.

The medical hydrogel of the present invention preferably is a sterilized vehicle for drug delivery to be implanted in a body. Also preferably, the gelatin or derivative thereof in the medical hydrogel of the present invention is of porcine, bovine, or piscine origin. The medical hydrogel of the present invention also preferably contains a water-soluble solvent together with water.

The hydrogel of the present invention is characterized in that a specific number of crosslinks per molecule is introduced by a chemical or physical means, based on a detailed analysis from a chemical and polymer scientific perspective of the properties of the inherently hydrated starting material, and correlation of the properties with the conditions required for specific applications.

The hydrogel of the present invention was achieved based on the first-time discovery of polymer scientific information not heretofore known and on an investigation of its function in view thereof. It has been thought up to now that the absorption properties, degradation characteristics, and physical properties of a hydrogel are determined almost entirely by its origin. Moreover, for hydrogels of natural origin, it is generally understood that all the properties are ultimately determined by the processing method, such as hydrolysis. For example, the properties of alginic acid, hyaluronic acid, dextran, and so forth, are determined by their source and method of processing. It is not possible to design those hydrogels that exhibit excellent solid state properties or those which exhibit both excellent excretability and appropriate viscosity.

The present inventor therefore carried out basic research into a method for preparing a hydrogel that can be used for implantation in a body and that would achieve a desired functionality in terms of physical and chemical properties, bioabsorbability, and drug delivery functionality. To date, medical hydrogels have been in frequent use mainly as wound dressing materials and are designed to have characteristics that provide a moist condition with the goal of wet healing and to have constant physical properties. For example, a material with desired physical properties has been selected to obtain an adhesion preventing material that can remain as a solid at the site of a surgical wound and provide sufficient covering and tissue separation for a prescribed period of time. These materials are designed to utilize the intrinsic properties of the materials. In contrast, the present inventor conducted detailed analysis of hydrogels with regard to their polymer scientific properties, and has elucidated for the first time the prerequisites to allow for implantation in a body and to achieve a desired functionality in terms of the physical and chemical properties, bioabsorbability, drug delivery, and so forth.

The present invention is based on the elucidation of the number of functional groups present on the polymer chain through a detailed analysis of gelatin and derivatives thereof, which are hydrogel starting materials, and on the discovery that the hydrogel can be endowed with specific physical and chemical functionalities by simply controlling the number of crosslinks when a crosslinking reaction takes place between those functional groups. Various specific functionalities are required of a medical hydrogel depending on its application and purpose, including drug delivery, a wound dressing material, an adhesion preventing material, and a separation film. For example, for an adhesion preventing material, the hydrogel must retain its shape for 4 days to provide a physical separation and then it must have a suitable absorbability to allow for rapid elimination from the site of application and excretion from the body. In the case of drug delivery applications, the hydrogel must be designed to have a prescribed release interval and shape stability during that interval. The present inventor carried out detailed investigations of such desired functionalities and elucidated the polymer scientific properties of hydrogels required for those functionalities. The present invention may of course be applied to every hydrogel without regard to the type or molecular weight of the polymer constituting the hydrogel and without regard to the crosslinking reaction method.

The use of the medical hydrogel according to the present invention enables the controlled release, from long-term to short-term depending upon the desired interval, of all types of drugs, for example, a low, intermediate, or high molecular weight drugs whose controlled release has heretofore been problematic, such as growth factors, cytokines, and peptides. Also according to the present invention, adhesion preventing materials and wound dressing materials may have a desired shape stability and may be used as a material for drug delivery.

### Brief Description Of The Drawings

Figure 1 shows the time course of degradation and elimination of a subcutaneously implanted crosslinked gelatin hydrogel (iodine isotope label); and
Figure 2 shows that the degradation rate of a subcutaneously implanted crosslinked gelatin hydrogel is proportional to the number of crosslinks in the gelatin molecule regardless of the crosslinking method or water content.

### Description Of The Preferred Embodiments

The hydrogel of the present invention is described in detail below.

When gelatin or a derivative thereof is used as a medical gel, the interval over which shape retention is required varies with the application, and as a consequence investigations were first carried out into how to adjust this interval. For example, application as a post-surgical adhesion preventing material in a body requires that the material remain at the site and retain its shape for four days after implantation, without in vivo degradation or destruction. In addition, once this interval has passed, rapid degradation and elimination are required. A wound dressing material for dermal regeneration in plastic surgery must maintain its shape over at least 7 days, with preventing exudate, if any, from flowing while keeping its moisture content. When a hydrogel is used as a drug delivery vehicle, a precise degradation characteristic is required that meets the desired delivery interval in correspondence to the intended application; for example, depending on the application, from about 2 days to 3 months may be required.

With respect to the above requirements, the present inventor has succeeded in elucidating for the first time the polymer scientific characteristics of gelatin and derivatives thereof that should be used as indices in order to achieve a desired objective. That is, the inventor has elucidated, among the chemical properties of gelatin, the type and number of side-chain functional groups present on the polymer chain and has discovered that the polymer scientific properties required for each individual application can be obtained by inducing a specific number of crosslinking reactions in a hydrogel of gelatin or a derivative thereof. The in vitro solubility and degradability of gelatin are well known. In general, gelatin is readily soluble in water and in vivo, resulting in a loss of its initial shape. Due to this nature, when gelatin is used as a drug delivery vehicle, it can hardly remain at the site of application for the required interval, resulting in elution and disappearance of the incorporated drug from the site. The present inventor discovered that the shape of the hydrogel can be stabilized by the introduction of crosslinking into the gelatin or derivative thereof and that the amount of residual gel in vivo is clearly governed by the number of crosslinks introduced into the hydrogel. Based on this mechanism, it was elucidated for the first time that the precisely controlled release of a drug and maintenance of the shape required for a specific goal or application can be achieved by selection of the number of crosslinks.

According to the present invention, the properties of the hydrogel can be controlled over a broad range, from a near-liquid hydrogel to a viscous hydrogel to a completely solid hydrogel, by a crosslinking reaction that utilizes a specific type and number of side chains. The present invention is based on the discovery that the number of crosslinks that closely corresponds to the required drug delivery interval, which for soft tissue is from 5 days to a maximum of 28 days and for hard tissue is from 5 weeks to a maximum of 12 weeks, is from 0.10/molecule to 50/molecule. The present invention makes it possible to carry out a complete quality design of a hydrogel, of which behavior in vivo has not been realized. In addition to the release interval, another requirement of a hydrogel is that it must be in a liquid state with a certain fluidity when delivered as a vehicle. In this case the required number of crosslinks is up to about 0.010. This means that, in accordance with the present invention, the required number of crosslinks has been found to be from 0.010/molecule to 50/molecule based on the discovery of the in vivo degradation behavior of crosslinked hydrogels, which permits unrestricted control. The number of crosslinks in the hydrogel can be calculated with the Flory equation using dynamic viscoelastic measurements with, for example, a rheometer, or the value of the initial elastic modulus from a tensile test instrument.

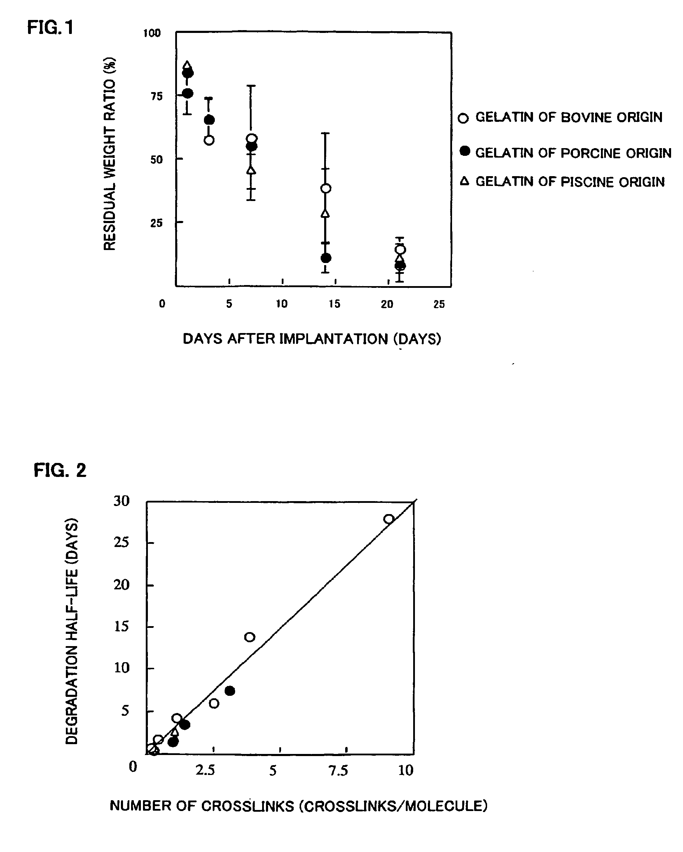

Figure 1 shows in vivo degradation behavior of the crosslinked hydrogel obtained by inducing a crosslinking reaction in a hydrogel of gelatin or a derivative thereof so as to give a specific number of crosslinks. The gelatin or derivative thereof (iodine isotope label) degrades with time and its residual radioactivity (corresponding to the residual hydrogel weight) decreases. The inventor has demonstrated for the first time that the hydrogel degradation rate in vivo is proportional simply to the number of crosslinks in the gelatin or gelatin derivative, without regard to the source of the gel, water content, or the crosslinking method as proposed before (Figure 2). That is, the in vivo degradation rate can be definitely controlled by introducing a specific number of crosslinks into the hydrogel. This findings enabled to prepare a carrier with a degradation rate that is precisely adapted to the interval required for drug release. The present invention elucidates the residual amount and kinetics of a gelatin hydrogel with a specific number of crosslinks containing basic fibroblast growth factor (iodine isotope label). Accordingly, the hydrogel can be designed by selecting the number of crosslinks that corresponds to the interval required for drug release.

In addition to the newly discovered knowledge described above, the conditions required for the in vivo delivery and controlled release of, for example, growth factors such as bFGF, HGF, PDGF, TGF, IGF, proteins such as BMP, cytokines, chemikines, monokines, as well as medium-sized and small molecules such as hormones, peptides, nucleic acids, polysaccharides, oligosaccharides, decoys, and siRNA were investigated. It was found that the required quantity for growth factors and nucleic acids is very small at 200 to 400 µg/3 mg gelatin, and that the drugs must be continuously administered over a long interval, for example, 2 to 4 weeks for soft tissue and 6 to 12 weeks for hard tissue. Thus, no effect is observed when a total amount is locally administered at once, which may lead to adverse side effects. This means that the delivery of the drugs requires a very strictly controlled release with regard to amount, interval, and the location in the tissue. The desired controlled release can be achieved according to the present invention with the use of a hydrogel in which a specific number of crosslinks has been introduced, that is, 0.1/molecule to 50/molecule. Furthermore, the hydrogel of the present invention prepared by selecting a specific number of crosslinks may be used not just as a controlled release carrier for drugs, but also as an adhesion preventing material and wound healing material that exhibit an excellent in vivo shape retention, stability, and functionality. Such hydrogels may also deliver, for example, preferred growth factors and/or nucleic acids at the site of application.

The content of all the patents and references explicitly cited in the Specification are hereby incorporated by reference in its entirety. In addition, the contents of the Specification and drawings of Japanese Patent Application 2005-36309, which is the priority application for the present application, is hereby incorporated by reference in its entirety.

Examples of the present invention are provided below, but the present invention is not limited to these examples.

### Example 1

Because it has been found that the number of crosslinks in a hydrogel contemplated for the drug release of a growth factor over 4 days is 1.0/molecule, a crosslinked hydrogel with such a property was prepared. An acidic gelatin (Nitta Gelatin Inc.) with a molecular weight of 100,000, that was obtained by the base treatment of collagen of bovine origin, was dissolved in water at 4.50 weight%, 2.5 mM glutaraldehyde was added, and let stand for 24 hours with cooling. The resulting hydrogel was soaked in an aqueous glycine solution at room temperature for 3 hours, then washed with distilled water and lyophilized for 24 hour. The viscoelasticity of the crosslinked hydrogel was measured and the number of crosslinks calculated from the Flory-Huggins equation was found to be 1.12/molecule.

In order to test the release behavior, the resulting hydrogel was impregnated with a phosphate-buffered physiological saline solution (pH 7.4) containing 3 micrograms of basic fibroblast growth factor (bFGF) labeled with radioactive iodine. The hydrogel was then implanted in the back of rats and the release was measured. The release period was 5.4 days, which was close to the desired interval.

### Example 2

Continuous drug release for 2 weeks is required for the drug delivery of growth factors for regeneration of soft tissues. It has been found that the preferred number of crosslinks in a hydrogel suitable for such an application is 6.0/molecule. Basic collagen of porcine tendon origin (Nitta Collagen Inc.) was treated with 10-fold aqueous ethylenediamine solution and neutralized with dilute hydrochloric acid to adjust to a weakly acidic state. At this point the amino group count was approximately 65/molecule and the carboxyl group count was approximately 125/molecule. Water-soluble carbodiimide was added at 2.2-times as the molar ratio with respect to the carboxyl groups, and let stand overnight at room temperature to obtain a cationized gelatin. The 8.8% aqueous solution of the cationized gelatin was stored overnight at 4 to 6°C to form a hydrogel, which was crosslinked by treatment with glutaraldehyde at a concentration of 0.25 microgram/mL in an acetone-dilute hydrochloric acid mixed solution at 4 to 6°C for 30 hours. The hydrogel was treated with an aqueous glycine solution, washed with distilled water, and lyophilized for 24 hours. It was then heated at 140°C for 24 hours in order to further develop the crosslinking. The number of crosslinks, as calculated with the Flory equation from the initial mechanical properties of the hydrogel, was 3.3/molecule prior to the heat treatment and 6.5/molecule after the heat treatment. The hydrogel was impregnated with bFGF and examined for the release rate as in Example 1. The release ratio was 15.5 days.

### Example 3

The regeneration of hard tissue generally takes 6 to 8 weeks, and the interval for the regeneration with the aid of a drug delivery of growth factor is also the same. It was determined that the number of crosslinks in a hydrogel that corresponds to the interval is 9/molecule. A hydrogel sheet obtained as in Example 1 was additionally heat treated for 24 hours at 140°C and then exposed twice to a gamma beam at 25 kGy to prepare a crosslinked hydrogel sheet. The number of crosslinks calculated from the initial mechanical properties was 10.8/molecule, which was almost equal to the desired value.

The hydrogel of the present invention is suitably used for medical applications, for example, drug delivery, adhesion prevention and wound healing utilizing its degradation behavior in a body.

## Claims

1. A substantially water-based hydrogel or a dried material thereof, wherein the hydrogel has at least 0.010 but no more than 50 crosslink points per molecule, and wherein the hydrogel is obtained by crosslinking a gelatin having 20 to 30 amino groups per molecule and the same or greater number of carboxyl groups per molecule or a gelatin derivative having 2 to 100 amino groups per molecule and 50 or more carboxyl groups per molecule.

2. The hydrogel or dried material thereof according to claim 1, wherein the gelatin or derivative thereof is crosslinked by one or more methods selected from chemical methods and physical methods.

3. The hydrogel or dried material thereof according to claim 1 or 2, wherein the gelatin or derivative thereof is crosslinked by a physical method and the hydrogel has a degree of crosslinking that varies in accordance with a distance from its surface.

4. The hydrogel or dried material thereof according to claim 1, 2, or 3, wherein the hydrogel is a sterilized vehicle for drug delivery to be implanted in a body.

5. The hydrogel or dried material thereof according to claim 1, 2, 3, or 4, wherein the gelatin or derivative thereof is of porcine, bovine, or piscine origin.

6. The hydrogel or dried material thereof according to claim 1, 2, 3, 4, or 5, wherein the hydrogel contains water and a water-soluble solvent other than water.

7. The hydrogel or dried material thereof according to any one of claims 1 to 6, wherein the hydrogel is a wound dressing material.

8. The hydrogel or dried material thereof according to any one of claims 1 to 6, wherein the hydrogel is an adhesion preventing material.

9. The hydrogel or dried material thereof according to any one of claims 1 to 6, wherein the hydrogel is a separation film.

10. The hydrogel or dried material thereof according to any one of claims 4 and 7 to 9 for delivering growth factors, proteins, cytokines, chemokines, monokines, hormones, peptides, nucleic acids, polysaccharides, oligosaccharides, decoys, and siRNA.

## Patentansprüche

1. Ein Hydrogel, das im Wesentlichen auf Wasser basiert, oder ein getrocknetes Material davon, wobei das Hydrogel mindestens 0,010, jedoch nicht mehr als 50 Vernetzungsstellen pro Molekül aufweist, und wobei das Hydrogel durch Vernetzen einer Gelatine, welche 20 bis 30 Aminogruppen pro Molekül und die gleiche Anzahl oder eine größere Anzahl an Carboxylgruppen pro Molekül aufweist, oder eines Gelatinederivats, welches 2 bis 100 Aminogruppen pro Molekül und 50 oder mehr Carboxylgruppen pro Molekül aufweist, erhalten wird.

2. Das Hydrogel oder das getrocknete Material davon gemäß Anspruch 1, wobei die Gelatine oder das Derivat davon durch ein oder mehr Verfahren, ausgewählt aus chemischen Verfahren und physikalischen Verfahren, vernetzt wird.

3. Das Hydrogel oder das getrocknete Material davon gemäß Anspruch 1 oder 2, wobei die Gelatine oder das Derivat davon durch ein physikalisches Verfahren vernetzt wird und das Hydrogel einen Vernetzungsgrad aufweist, der in Abhängigkeit von einem Abstand von seiner Oberfläche variiert.

4. Das Hydrogel oder das getrocknete Material davon gemäß Anspruch 1, 2 oder 3, wobei das Hydrogel ein sterilisiertes Vehikel zur Medikamentenverabreichung ist, das in einen Körper implantiert werden soll.

5. Das Hydrogel oder das getrocknete Material davon gemäß Anspruch 1, 2, 3 oder 4, wobei die Gelatine oder das Derivat davon porcinen, bovinen oder piscinen Ursprungs ist.

6. Das Hydrogel oder das getrocknete Material davon gemäß Anspruch 1, 2, 3, 4 oder 5, wobei das Hydrogel Wasser und ein wasserlösliches Lösungsmittel, das von Wasser verschieden ist, enthält.

7. Das Hydrogel oder das getrocknete Material davon gemäß einem der Ansprüche 1 bis 6, wobei das Hydrogel ein Wundauflagematerial ist.

8. Das Hydrogel oder das getrocknete Material davon gemäß einem der Ansprüche 1 bis 6, wobei das Hydrogel ein Material zur Vermeidung von Anhaftung ist.

9. Das Hydrogel oder das getrocknete Material davon gemäß einem der Ansprüche 1 bis 6, wobei das Hydrogel eine Trennfolie ist.

10. Das Hydrogel oder das getrocknete Material davon gemäß einem der Ansprüche 4 und 7 bis 9 zur Verabreichung von Wachstumsfaktoren, Proteinen, Cytokinen, Chemokinen, Monokinen, Hormonen, Peptiden, Nukleinsäuren, Polysacchariden, Oligosacchariden, Decoys und siRNA.

## Revendications

1. Hydrogel essentiellement à base d'eau ou matière sèche de celui-ci, dans lequel l'hydrogel a au moins 0,010 mais pas plus de 50 points de réticulation par molécule, et dans lequel l'hydrogel est obtenu par réticulation d'une gélatine ayant 20 à 30 groupes amino par molécule et le nombre identique ou supérieur de groupes carboxyle par molécule ou d'un dérivé de gélatine ayant 2 à 100 groupes amino par molécule et 50 groupes carboxyle ou plus par molécule.

2. Hydrogel ou matière sèche de celui-ci selon la revendication 1, dans lequel la gélatine ou son dérivé est réticulé par un ou plusieurs procédés choisis parmi des procédés chimiques et des procédés physiques.

3. Hydrogel ou matière sèche de celui-ci selon les revendications 1 ou 2, dans lequel la gélatine ou son dérivé est réticulé par un procédé physique et l'hydrogel a un degré de réticulation qui varie en fonction d'une distance par rapport à sa surface.

4. Hydrogel ou matière sèche de celui-ci selon les revendications 1, 2 ou 3, dans lequel l'hydrogel est un véhicule stérilisé pour l'administration de médicaments destiné à être implanté dans un corps.

5. Hydrogel ou matière sèche de celui-ci selon les revendications 1, 2, 3 ou 4, dans lequel la gélatine ou son dérivé est d'origine porcine, bovine ou ichtyique.

6. Hydrogel ou matière sèche de celui-ci selon la revendication 1, 2, 3, 4 ou 5, dans lequel l'hydrogel contient de l'eau et un solvant hydrosoluble autre que l'eau.

7. Hydrogel ou matière sèche de celui-ci selon l'une quelconque des revendications 1 à 6, dans lequel l'hydrogel est un matériau pour pansement de plaies.

8. Hydrogel ou matière sèche de celui-ci selon l'une quelconque des revendications 1 à 6, dans lequel l'hydrogel est un matériau anti-adhésif.

9. Hydrogel ou matière sèche de celui-ci selon l'une quelconque des revendications 1 à 6, dans lequel l'hydrogel est un film de séparation.

10. Hydrogel ou matière sèche de celui-ci selon l'une quelconque des revendications 4 et 7 à 9 pour administrer des facteurs de croissance, des protéines, des cytokines, des chémokines, des monokines, des hormones, des peptides, des acides nucléiques, des polysaccharides, des oligosaccharides, des leurres et des siARN.
